Europäisches Patentamt

European Patent Office

Office européen des brevets

(11) Veröffentlichungsnummer: **0 279 867**
**A2**

## (12) EUROPÄISCHE PATENTANMELDUNG

(21) Anmeldenummer: 87100286.1

(22) Anmeldetag: 12.01.87

(51) Int. Cl.⁴ **A61K 31/355** , A61K 31/165 , A61K 31/19

Die Bezeichnung der Erfindung wurde geändert (Richtlinien für die Prüfung im EPA, A-III, 7.3).

(43) Veröffentlichungstag der Anmeldung:
**31.08.88 Patentblatt 88/35**

(84) Benannte Vertragsstaaten:
**AT BE CH DE ES FR GB GR IT LI LU NL SE**

(71) Anmelder: **EFEKA Friedrich & Kaufmann GmbH & Co KG**
**Siemensstrasse 1**
**D-3004 Isernhagen 1(DE)**

(72) Erfinder: **Blankenhorn, Gunter, Dr.**
**Lahriede 21**
**DE-3004 Isernhagen 1(DE)**
Erfinder: **Fischer, Ingo, Dr.**
**Eichenkampf 5**
**D-3004 Isernhagen 4(DE)**

(74) Vertreter: **Wissbar, Ralf**
**Mohnhof 19A**
**D-2050 Hamburg 80(DE)**

(54) **Verwendung von Vitamin-E und Vitamin-E-Kombination mit nichtsteroidalen Antirheumatika und Schmerzmittel zur Behandlung von rheumatischen Erkrankungen.**

(57) Die Erfindung besteht in der Erkenntnis der Eignung von Vitamin E zur Behandlung rheumatischer Erkrankungen. In mehreren Klinischen Studien wurde bei Patienten mit rheumatischen Beschwerden unterschiedlicher Genese nach Therapie mit Vitamin E eine Entzündungshemmung, Verminderung der Schwellung und Reduktion der Schmerzen ohne Auftreten von Nebenwirkungen beobachtet. Zudem konnten der zuzätzliche Bedarf an Analytical/Antiphlogistika eingeschränkt werden.

Bei der beanspruchten Indikation können Vitamin E und Vitamin E-Kombinationen in ovaler fester und flüssiger Darreichungsform sowie parenteral und topisch angewendet werden und sollten in der Dosierung von Vitamin E 300 I.E. nicht unter-und 1500 I.E. nicht überschreiten.

EP 0 279 867 A2

## Neue Verwendung von alpha Tocopherol und alpha-Tocopherolderivaten

Gegenstand der vorliegenden ist neue Verwendung von alpha-Tocopherol und alpha-Tocopherol-Derivaten - im folgenden werden beide Gruppen in bekannter Weise Vitamin E genannt.

Vitamin E ist bekannt als Antioxidant und Schutzvitamin für Phosphol der Zellmembran. Es hält die Permeabilität und Stabilität der Zellmembran aufrecht; Lucy J.A., Annals N.Y. Acadmy of Sience 203, S. 4 (1972). Es ist weiterhin bekannt, daß Vitamin E eine membranabdichtende Wirkung besitzt; Mittelbach F und Bodechtel G., Münchner Medizinische Wochenzeitschrift 110/36, S. 1988-1993 (1968). Bei Erythrozyten, den einfachsten Zellen des menschlichen Körpers, wurde festgestellt, daß Vitamin E eine Schutzwirkung für die Zellmembran darstellt. In Tierversuchen und beim Menschen wurde bewiesen, daß Anämie das erste Anzeichen von Vitamin-E-Mangel ist. Bei Gabe von hohen Vitamin-E-Dosen normalisiert sich die Hämolyse der Erythrozyten; vgl. Villism J. Darbey, Vitamin.Horm. 26/50, S. 685 -704 (1968) und Phelps D.L., Pediatrics 63/6, S. 933 -935 (1979). Aus diesen Literaturstellen geht hervor, daß bei Gabe von 200 bis 800 mg Vitamin E oral für einen Zeitraum von 1 bis 4 Tagen die Hämolyse der Erythorzyten signifikant verbessert wird im Vergleich zu Patienten mit Vitamin-E-Mangel.

Vitamin E ist weiterhin verwendet worden zur Behandlung der Sichelzellenanämie in einem Zeitraum von 6 bis 35 Wochen; vgl. Natt CL., Am. J. Clin. 33, S. 968 - 971 (1980); Natt. Cl., Am. J. Clin Nutr. 32, S. 1359 - 1362 (1979); Gawlik G,M, Fed. Proc. 35/3, S.252 (1976) und Gorash L., Bieri J.G. et al. Univ. Conn. Framington, GT.

Weiterhin ist bekannt, daß 750 mg Vitamin E täglich in einem Zeitraum von 3 bis 6 Monaten erfolgreich bei Thalassamie-Patienten eingesetzt wurde, wobei eine Normalisierung der Hämolyse der Erythrozyten beobachtet wurde; vgl. Kahane I., ISR. J. Med. 12/1, S 11 - 15 (1976).

Erfolgreich eingesetzt wurde Vitamin E weiterhin bei Patienten mit akuter Hepatitis und alkoholischer Hepatitis, die einen Mangel an Vitamin E im Serum haben; vgl. Yoshiakawa T. Takemura S. Kato H. et al., Japan J. Gastrovent, 74/7, S. 732 - 739 (1977). Schließlich wurde Vitamin E bei Patienten mit Eisenmangelanämie eingesetzt und bewirkte während eines Zeitraumes von 4 bis 8 Wochen eine Verbesserung bzw. Normalisierung des Lipidmetabolismus im Knochenmark; vgl.. Takoshi Itaga, Central Clinical Laboratory Nagasaki University of Medicine, Japan.

Erfolgreich wurden Vitamin-E-Mangelzustände bei Malabsorptionsstörungen, zystischer Fibrose, Retrolentale Fibroplasie, Frühgeborenen-Anämie, chr. Steatorrhoe durch parenterale oder orale Gabe von Vitamin E behandelt (I. Elmadfa, W.Bosse, Vitamin E, Wiss, Verlagsgesellschaft, Stuttgart 1985).

Es wurde überrachenderweise gefunden, daß Vitamin E darüber hinaus als aktiver Stoff, als Wirkstoff, für die therapeutische Behandlung rheumatischer Erkrankungen geeignet ist. Dieser neue Indikationsbereich war aufgrund des bisherigen Wissensstandes nicht vorherzusehen und eröffnet ein neues, breites Anwendungsfeld für Vitamin E. Es wurde gefunden, daß dieser Wirkstoff sich zur Herstellung von Antiphlogistika bzw. Antirheumatika verwenden läßt.

Bei rheumatischen Erkrankungen handelt es sich um Schwellungen, Schmerzen und Funktionseinschränkungen in den Bewegungsorganen. Pathologisch gesehen, wird Bindegewebe zerstört. Entsprechend der Anteile des Bewegungsapparates lassen sich folgende rheumatische Erkrankungen unterscheiden: Gelenkerkrankungen, Wirbelsäulenerkrankungen, Weichteilerkrankungen und Knochenerkrankungen (Mathies, H., Rheuma, Gustav Fischer Verlag, Stuttgart, New York 1983).

Nach diagnostisch-therapeutisch-praktischen Gesichtspunkten werden unterschieden: entzündlicher Rheumatismus, degenerativer Rheumatismus und extraartikulärer Rheumatismus (Pschyrembel W., Klinisches Wörterbuch, Walter de Gruyter, Berlin, New York 1982).

Eine erfolgreiche Therapie gegen rheumatische Erkrankungen ist bis heute nicht bekannt. Die antirheumatischen Medikamenten wirken meist unbefriedigend und müssen langfristig angewandt werden. Außer Chloroquin, Gold, D-Penicillamin, Kortisionen und anderen sympthomatisch wirkenden Antirheumatika wurden auch Immunsuppressiva eingesetzt (Mathies, H., 1983; Butler, R.C: und Goddard, D.H., Controversy in the treatment of rheumatoid arthritis, Lance ii, 278 -179, 1985).

Diese Therapien sind heute Standard, sind jedoch mit zum Teil erheblichen Nebenwirkungen verbunden. Im Rahmen eigener in vitro-Versuche und klinischer Prüfungen konnte bei der Behandlung verschiedener rheumatischer Erkrankungen unerwartet gefunden werden, daß sowohl eine natürliche ( -alpha-Tocopherole) als auch eine synthetische d, (l-alpha-Tocpoherole) Vitamin E enthaltende Präparation zur Hemmung der Entzündung, Verminderung der Schwellung und Reduktion der Schmerzen führt. Dadurch wird die Beweglichkeit der Patienten verbessert. Auf die zusätzliche Einnahme von Schmerzmitteln während der Vitamin-E-Therapie konnte verzichtet oder die Dosis stark reduziert werden. Gerade bei einer längeren Therapie werden so die Nebenwirkungen der üblichen Antirheumatika reduziert und die Sicherheit für den Patienten erhöht. Dieses Ergebnis überrascht, da die bisher klinisch gesichteten Wirkungen von Vitamin E dies nicht vermuten ließen.

Es wurde ferner gefunden, daß ausreichend dosiertes Vitamin E in Kombination mit niedrig dosierten nichtsteroidalen Antirheumatika und Schmerzmitteln wie Diclofenac, Ibuprofen, Paracetamol die Wirksamkeit erheblich erhöht, ohne das Nebenwirkungsrisiko zu erhöhen. Diese Ergebnisse waren nicht vorhersehbar und ermöglichen eine Therapie, bei der ein Teil des chemischen Wirkstoffs durch einen Naturstoff ersetzt wird, der sich in kleinen Mengen obendrein praktischn in jeder Körperzelle befindet.

Zweckmäßig für die Wirksamkeit von Vitamin-E-Präparationen als Wirkstoff für die erfindungsgemäße Verwendung, auch in Form von Kombinationtn von Vitamin E mit Schmerzmitteln und nichtsteroidalen Antirheumatika ist vor allem eine ausreichende Dosierung, die mindestens 100 I.E. betragen sollte. Es wurde gefunden, daß zu niedrige Dosierungen, beispielsweise so geringe Mengen Vitamin E wie etwa 10-80 I.E., in der Regel für die Behandlung rheumatischer Erkrankungen wirkungslos bleiben.

Als Vitamin E können sowohl Ester aus natürlicher oder synthetischer Herkunft als auch das freie alpha-Tocopherol verwendet werden. Es versteht sich, daß alle für die erfindungsgemäße Verwendung einsetzbaren alpha-Tocopherol-derivate physiologisch gut verträgliche Stoffe sind.

Bei der erfindungsgemäßen Verwendung lassen sich zusätzlich die üblichen Träger-und Hilfsstoffe mitbenutzen.

Vitamin E und Vitamin-E-Kombination können bei der beanspruchten Indikation wie folgt dosiert werden. Die Substanzen können sowohl in einer oralen festen und flüssigen Darreichungsform als auch parenteral und topisch angewendet werden. Die Tagesdosis von Vitamin E sollte 300 I.E. in der Kombination und in der Einzelanwendung nicht unterschreiten und 1.500 I.E. nicht überschreiten. Die Tagesdosis kann auf einmal oder aufgeteilt aud 3 Einzeldosen gegeben werden. Zur Steigerung der Effektivität können zusätlich alle in der Therapie bisher verwendete Antirheumatika gegeben werden.

Beispiele für die Zusammensetzung:

Beispiel 1 : Monopräparat :

Kapsel aus
400 mg = 544 I.E. d-alpha-Tocopherolacetat
70 mg Sojalecithin (Hilfsstoff)
sonstige Hilfs-und Farbstoffe

Beispiel 2: Monopräparat:

Kapsel aus
125 mg = 125 I.E. d,l-alpha Tocopherolacetat
30 mg Sojalicitin (Hilfsstoff)
sonstige Hilfs-und Farbstoffe

Beispiel 3: Kombination:

Kapsel aus
100 mg = 100 I.E. d,l-alpha-Tocopherolacetat
30 mg Weizenkeimöl (Hilfsstoff)
25 mg (50 mg) Diclofenac-Natrium
sonstige Hilfs-und Farbstoffe

Beispiel 4: Kombination:

Tabletten aus
300 mg = 300 I.E. d,l-alpha Tocopherolsuccinat
300 mg Paracetamol
sonstige Hilfs-und Farbstoffe

Beispiel 5: Kombination:

Kapseln aus
200 mg = I.E. d,l-alpha-Tocopherolacetat
400 mg Ibuprofen
sonstige Hilfs-und Farbstoffe


Beispiel 6: Parentale Form (Ampullen):

220 mg : 300 I.E. d-alpha Tocopherolacetat
Miglyol ad 2 ml
Hilfsstoffe, Stabiolisatoren


Beispiel 7: Topische Form Salbe:

100 g Salbe enthalten 10 g - 10 000 I.E. d,l-alpha Tocopherolacetat
Salbengrundlage.


Beispiele für Indikationen:

1. in vitro Daten

Leukozyten spielen im entzündlichen rheumatischen Prozeß eine wesentliche Rolle. Die Infiltration von polymorphkernigen Granulozyten und das Maß Phagozytose sind Kriterien für eine rheumatische Entzündung. Von einer entzündungshemmenden Substanz muß heute gefordert werden, daß sie die Phagozyten und die Chemotaxe hemmt.

Es wurde gefunden, daß Vitamin E die Chemotaxe und Phagozythose neutrophiler Human-Granulozyten dosisabhängig hemmt.

Zur Messung wurden neutrophile Granulozyten in einem Percoll-Gradienten isoliert und in einem Medium suspendiert. Die Zellsuspension wurde in Boydenkammern eingesetzt und die Chemotaxis gegen eine Tripeptid gemessen. Die Messung der Phagozytenhemmung wurde nach Start durch opsonisiertes Zymosan mit einem Luminometer gemessen.


2. Klinische Untersuchungen

2.1. Aktivierte Arthrosen

50 Patienten mit aktivierten Arthrosen wurden in 6 Praxen (Ärzte für Orthopädie und Allgemeinmedizin) randomisiert über mehrere Wochen mit einem hochdosierten Vitamin-E-Präpart (400 I.E. d-alpha-Tocopherolacetat) oder identisch aussehenden Placebo-Kapseln behandelt.

Die statistische Auswertung dieser kontrollierten Doppelblindstudie zeigte, daß Vitamin E hinsichtlich der untersuchten Kriterien Schmerz( Ruheschmerz, Bewegungsschmerz, Druckschmerz), Schmerzmitteleinsparung und dem pauschalen Arzturteil gut wirksam und insbesondere der Placebowirkung signifikant bis hochsignifikant überlegen war.

Vitamin E zeigte zusätzlich eine gute Wirkung in bezug auf die Verbesserung bestehender Bewegungseinschränkung, war hierbei jedoch Placebo nicht signifikant überlegen. Hinsichtlicher Art und Häufigkeit beobachteter Nebenwirkungen unterscheiden sich die Vitamin E-und die Placebogruppe kaum voneinander. Durch diese Studie wird die antiphlogistische Wirksamkeit von Vitamin E klinisch belegt. Die unter Vitamin-E-Behandlung deutliche Schmerzmitteleinsparung gewinnt unter dem Gesichtpunkt der Erhöhung der Atzneimittelsicherheit große Bedeutung bei der Behandlung entzündlich rheumatischer Erkrankungen

## 2.2 Morbus Bechterew

24 Patienten mit Spodylitis ankylosans ( Morbus Bechterew) im Stadium I bis II nach Hartl wurden radomisiert doppelblind über 12 Wochen mit einem hochdosierten Vitamin-E-Präparat ( 544 I.E. d-alpha Tocopherolacetat ) oder identisch aussehenden Diclofenac-Kapseln ( 50 mg Diflofenac-Natrium ) behandelt. Die statistische Auswertung und der interindividuelle Vergleich zeigte, daß hinsichtlich der Veränderungen objektiver ( Schober BWS und LWS, Atembreite, Fingerbodenabstand ) und subjektiver Bewertungskriterien ( Morgensteifigkeit, Ruheschmerz Tag/Nacht, Bewegungsschmerz, Allgemeinbefinden ) sowie hinsichtlich gängiger Laborparameter ( Differentialblutbild, verschiedene Entzündungskriterien bzw. Serumenzyme, u.a. ) zwischen den beiden Versuchsgruppen im Therapieverlauf kein statistisch signifikanter Unterschied festzustellen war.

Mit der vorliegenden Studie konnte die antiphlogistische Wirksamkeit von hochdosiertem Vitamin E im Vergleich zu Diclofenac bei Frühstadien des Morbus Bechterew nachgewiesen werden. Die orale Antioxidanstherapie mit Vitamin E ist also einer herkömmlichen Behandlung mit nichtsteroidalen Antirheumatika ( Diclofenac ) hinsichtlich des Wirkungseintritts und des Wirkungsausmaßes gleichzusetzen.

## 2.3 Tendophatien

7 Fälle mit gesicherter Diagnose ( Epikondylitis, Tendovaginitis ) wurden über 6 Wochen mit Vitamin E behandelt.

Behandlungsschema:

1.Woche 1125 I.E. Vitamin-E-Acetat/Tag
2.Woche 750 I.E. Vitamin-E-Acetat/Tag
3.-6.Woche 375 I.E. Vitamin-E-Acetat/Tag

Nach 6 Wochen waren 4 Fälle geheilt, in 2 Fällen war das Therapieergebnis nach Ansicht der Ärzte befriedigend, in einem Fall war das Therapieergebnis nicht befriedigend. Der Verbrauch an zusätzlichen nichtsteroidalen Antirheumatika und Schmerzmittel konnte im Durchschnitt auf die Hälfte reduziert werden.

## 2.4 Periarthritis

9 Fälle mit Periarthritis wurden über 6 Wochen mit Vitamin E behandelt.

Behandlungsschema:

1.Woche 1125 I.E. Vitamin-E-Acetat/Tag
2.Woche 750 I.E. Vitamin-E-Acetat/Tag
3.-6.Woche 375 I.E. Vitamin-E-Acetat/Tag

Nach 6 Wochen waren 6 Fälle geheilt, in 2 Fällen wurde das Therapieergebnis als gut bezeichnet, in 1 Fall war das Therapieergebnis noch nicht befriedigend.

## 2.5 Chronische Polyarthritis

Einzelfallstudien :

5 Patienten mit gesicherter chronischer Polyarthritis wurden nach einer 'wash out' Periode mit 1632 I.E. d-$\alpha$-Tocopherolacetat (3 x 1 Kapel à 544 I.E. pro Tag) über einen Zeitraum von 3 Wochen behandelt. Die Patienten standen während der gesamten Zeit unter ärztlicher Kontroller (Klinik).

Alle Patienten erfüllten mindestens 7 von 11 ARA-Kriterien zur Charakterisierung der PCP. Weder der Arzt noch der Patient wußten, ob die Therapie mit einer Vitamin E-Medikation oder einer Standard-Antirheumatikum-Medikation durchgeführt wurden (Doppelblind-Studie).

Folgende Parameter werden zur Beurteilung des Therapieerfolgs herangezogen:

5

1. Schwellung: Summe aus der Beurteilung von 29 Einzel-Gelenk-Betrachtungen (0 = keine Schwellung, 3 = sehr starke Schwellung)
2. Gehzeit: in Minuten
3. Schmerzen: Schmerzskala nach Littich (0 = keine Schmerzen, 10 = sehr starke Schmerzen)
4. Morgensteifigkeit der Gelenke: in Minuten
5. Griffstärke der Hände: in kpa (Vigometer)
6. Blutsenkung BSG: mm Hg/h
7. Eisen im Serum: $\mu$g/de
8. Blutbild:

Monozyten %
Lymphozythen %
Neutrophile %
9. Protein-Elektrophorese:
Globuline %

## Fall 1

| Patient: G. E. | Alter: 53 | Geschlecht: w |
|---|---|---|

|  | vor der Behandlung | nach der Behandlung |
|---|---|---|
| Schmerzen | 8 | 2 |
| Gelenk-Schwellung | 24 | 7 |
| Morgensteifigkeit | 45 | 10 |
| Griffstärke |  |  |
| Hand links | 38 | 53 |
| Hand rechts | 46 | 58 |
| Maximale Gehzeit | 15 | 60 |
| Laborwerte (Entzündungsparameter): |  |  |
| BSG | 30 | 21 |
| $\alpha_2$ Globulin % | 10,4 | 3,7 |
| Rheumafaktor (Latex): |  | neg. |
| Pauschales Arzturteil: |  | gebessert, Schmerzen, Schwellungen, Morgensteifigkeit sehr stark gebessert |

**Fall 2**

| Patient: A. H. | Alter: 64 | Geschlecht: W |
|---|---|---|
| | vor der Behandlung | nach der Behandlung |
| Schmerzen | 8 | 2 |
| Gelenk-Schwellungen | 14 | 13 |
| Morgensteifigkeit | 135 | 30 |
| Griffstärke | | |
| Hand links | 17 | 21 |
| Hand rechts | 13 | 17 |
| Maximale Gehzeit | 10 | 60 |
| Laborwerte (Entzündungsparameter): | | |
| Neutrophile Granulozyten | 70 | 60 |
| Monozyten | 7 | 5 |
| BSG | 29 | 26 |
| Rheumafaktor(Latex): | neg. | |
| Pauschales Arzturteil: | | gebessert, Schmerzen und Morgensteifigkeit stark gebessert |

Fall 3

Patient: L. S.  Alter: 55  Geschlecht: w

|  | vor der Behandlung | nach der Behandlung |
|---|---|---|
| Schmerzen | 5 | 2 |
| Gelenk-Schwellungen | 21 | 7 |
| Morgensteifigkeit | 75 | 0 |
| Griffstärke | | |
| Hand links | 33 | 27 |
| Hand rechts | 43 | 33 |
| Maximale Gehzeit | 60 | 60 |
| Laborwerte (Entzündungsparameter): | | |
| ß-Globulin | 11 | 10,3 |
| γ-Globulin | 20,5 | 18,9 |
| Eisen | 43 | 67 |
| Rheumafaktor (Latex): | positiv | |
| Pauschales Arzturteil: | | gebessert, Schwellungen und Morgensteifigkeit sehr stark gebessert |

## Fall 4

| Patient: J. B. | Alter: 65 | Geschlecht: w |
| --- | --- | --- |
| | vor der Behandlung | nach der Behandlung |
| Schmerzen | 8 | 3 |
| Gelenk-Schwellungen | 25 | 14 |
| Morgensteifigkeit | 240 | 20 |
| Griffstärke | | |
| Hand links | 19 | 35 |
| Hand rechts | 40 | 53 |
| Maximale Gehzeit | 10 | 20 |
| Laborwerte (Entzündungsparameter): | | |
| Neutrophile Granulozyten | 63 | 58 |
| Monozyten | 4 | 3 |
| BSG | 34 | 23 |
| Rheumafaktor (Latex): | positiv | |
| Pauschales Arzturteil: | | gebessert, Schmerzen, Schwellungen und Morgensteifigkeit sehr stark gebessert |

## Fall 5

| Patient: M. K. | Alter: 67 | Geschlecht: w |
| --- | --- | --- |
| | vor der Behandlung | nach der Behandlung |
| Schmerzen | 9 | 4 |
| Gelenk-Schwellungen | 57 | 16 |
| Morgensteifigkeit | 45 | 2 |
| Griffstärke | | |
| Hand links | 19 | 53 |
| Hand rechts | 45 | 48 |
| Maximale Gehzeit | 15 | 30 |
| Laborwerte (Entzündungsparameter): | | |
| Neutrophile Granulozyten | 73 | 64 |
| Monozyten | 9 | 7 |
| BSG | 28 | 11 |
| Eisen | 30 | 60 |
| $\alpha_1$-Globulin | 5,2 | 3,6 |
| $\alpha_2$-Globulin | 12,4 | 11,0 |
| ß-Globulin | 12,7 | 11,0 |
| Rheumafaktor (Latex): | negativ | |
| Pauschales Arzturteil: | | gebessert, Schmerzen, Schwellungen, Morgensteifigkeit sehr stark gebessert |

### 2.6 Morbus Scheuermann

31 meist jugendliche Patienten erhielten über 6 Wochen eine Vitamin-E-Präparation.
Die Dosierung betrug:
1. Tag 1125 I.E. d,l-alpha Tocopherolacetat
2. - 6. Tag 750 I.E. Tocopherolacetat

7. - 42. Tag 375 I.E. Tocopherolacetat

Die Auswertung der multicentrisch angelegten Studie ergab, daß Vitamin E in 71% der Fälle gute und in 16% der Fälle befriedigende Ergebnisse hinsichtlich der Wirksamkeit bei hervorragender Verträglichkeit zeigte. Die Beweglichkeit der Patienten wurde erheblich verbessert und die Häufigkeit auftretender Schmerzen stark reduziert. Nebenwirkungen wurden nicht beobachtet.

**Ansprüche**

1. Verwendung von alpha-Tocopherol und Derivaten dieses Stoffes als Wirkstoff für die Herstellung eines Arnzeimittels zur Behandlung rheumatischer Erkrankungen.

2. Verwendung nach Anspruch 1 zur Behandlung von entzündlichen und extraartikulärem Rheumatismus, im besonderen zur Behandlung von
- Aktivierten Arthrosen
- Morbus Bechterew
- Tendopathien (Epikondylitis, Tendovaginitis)
- Periarthritis
- Chronischer Polyarthritis
- Morbus Scheuermann

3. Verwendung nach Anspruch 1 und 2 in Kombination mit an sich bekannten anderen Antirheumatika und Schmerzmitteln, im besonderen Kombinationen mit Diclofenac-Natrium, Ibuprofen, Paracetamol.

4. Verwendung nach einem der Ansprüche 1 bis 3 in Form von Präparationen in einer oralen, intramuskulären intravenösen, intraarikulären oder topischen Applikationsform.

5. Verwendung nach einem der Ansprüche 1 bis 4, dadurch gekennzeichnet, daß die verabreichte Einzelform 100 bis 1000 Internationale Einheiten alpha-Tocopherol aufweist.